(19) Europäisches Patentamt
European Patent Office
Office européen des brevets

(11) Veröffentlichungsnummer: **0 104 550**
**A2**

(12) # EUROPÄISCHE PATENTANMELDUNG

(21) Anmeldenummer: 83109105.3

(22) Anmeldetag: 15.09.83

(51) Int. Cl.³: C 07 D 491/04
C 07 D 498/14, C 07 D 491/14
A 61 K 31/445

(30) Priorität: 23.09.82 DE 3235221

(43) Veröffentlichungstag der Anmeldung:
04.04.84 Patentblatt 84/14

(84) Benannte Vertragsstaaten:
AT BE CH DE FR GB IT LI NL SE

(71) Anmelder: BAYER AG
Konzernverwaltung RP Patentabteilung
D-5090 Leverkusen 1 Bayerwerk(DE)

(72) Erfinder: Seidel, Wolfgang, Dr.
Pahlkestrasse 3
D-5600 Wuppertal 1(DE)

(72) Erfinder: Kazda, Stanislav, Dr.
Gellertweg 18
D-5600 Wuppertal 1(DE)

(72) Erfinder: Knorr, Andreas, Dr.
Pahlkestrasse 15
D-5600 Wuppertal 1(DE)

(54) **Ringverbrückte 1,4-Dihydropyridine, Verfahren zu ihrer Herstellung und ihre Verwendung als Arzneimittel.**

(57) Die Erfindung betrifft neue, ringverbrückte 1,4-Dihydropyridine der allgemeinen Formel I

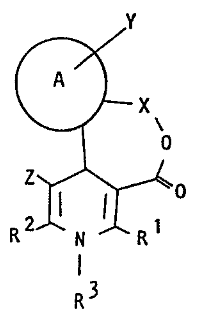

(I)

in welcher 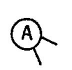 ,

Y, X, Z, $R^1$, $R^2$ und $R^3$ die in der Beschreibung angegebene Bedeutung besitzen, Verfahren zu ihrer Herstellung sowie ihre Verwendung in Arzneimitteln, insbesondere kreislaufbeeinflussenden Arzneimitteln.

EP 0 104 550 A2

BAYER AKTIENGESELLSCHAFT 5090 Leverkusen, Bayerwerk

Zentralbereich
Patente, Marken und Lizenzen KS/Mt-c

Ringverbrückte 1,4-Dihydropyridine, Verfahren zu ihrer Herstellung und ihre Verwendung als Arzneimittel.

Die Erfindung betrifft neue, ringverbrückte 1.4-Dihydropyridine, Verfahren zu ihrer Herstellung sowie ihre Verwendung in Arzneimitteln, insbesondere kreislaufbeeinflussenden Arzneimitteln.

Es ist bereits bekannt geworden, daß man 1,4-Dihydro-2,6-dimethyl-4-phenyl-pyridin-3,5-dicarbonsäurediethylester erhält, wenn man Benzylidenacetessigsäureethylester mit ß-Aminocrotonsäureethylester oder Acetessigsäureethylester und Ammoniak umsetzt (E. Knoevenagel, Ber. dtsch. chem. Ges. 31, 743 (1898)).

Weiterhin ist bekannt, daß bestimmte 1.4-Dihydropyridine interessante phamakologische Eigenschaften aufweisen und insbesondere als kreislaufbeeinflussende Mittel eingesetzt werden (F. Bossert, W. Vater, Naturwissenschaften 58, 578 (1971), DOS 2 117 571).

Hingegen sind die im folgenden beschriebenen ringverbrückten Dihydropyridine neu.

Die Erfindung betrifft neue 1.4-Dihydropyridine der allgemeinen Formel I

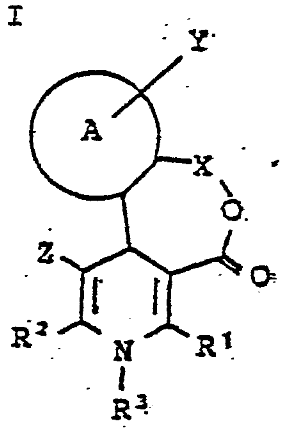

(I),

in welcher

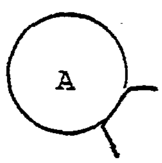 für Aryl, Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Chinolyl, Isochinolyl, Indolyl, Benzimidazolyl, Benzoxadiazolyl, Chinazolyl oder Chinoxalyl steht,

Y für einen oder zwei gleiche oder verschiedene Substituenten aus der Gruppe Alkyl, Alkenyl, Alkinyl, Alkoxy, Alkenoxy, Aryloxy, Aralkoxy, Alkinoxy, Phenyl, Halogen, Alkylen, Dioxyalkylen, Trifluormethyl, Trifluormethoxy, Nitro, Cyano, Azido, Carboxy, Hydroxy, Amino, Alkylamino, Halogenalkoxy, Carbalkoxy,

Carbonamido, Sulfonamido, $SO_m$-Alkyl, $SO_m$-Aryl oder $SO_m$-Aralkyl (m=0,1,2) steht,

X für eine geradkettige oder verzweigte Alkylkette mit bis zu 20 Kohlenstoffatomen steht, die mit 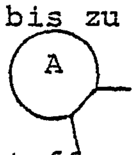 direkt oder über ein Sauerstoff-, Schwefel- oder Stickstoffatom verknüpft ist und die gegebenenfalls durch ein oder mehrere Sauerstoffatome, Stickstoffatome oder $-\overset{O}{\overset{\|}{C}}-O-$ Gruppen unterbrochen sein kann, wobei die oben genannten Stickstoffatome durch H, Alkyl, Acyl oder Benzyl substituiert sein können,

Z a) für die Gruppe $-COOR^4$ steht, wobei $R^4$ für einen geradkettigen, verzweigten oder cyclischen, gesättigten oder ungesättigten Kohlenwasserstoff steht, der durch Sauerstoff, Schwefel oder die $-SO_2$-Gruppe in der Kette unterbrochen sein kann, und der gegebenenfalls substituiert ist durch Halogen, Cyano, Pyridyl, Phenyl, Phenoxy, Phenylthio oder Phenylsulfonyl, wobei die Phenylgruppen ihrerseits substituiert sein können durch Halogen, Cyano, Dialkylamino, Alkoxy, Alkyl, Trifluormethyl oder Nitro, oder

wobei der Kohlenwasserstoffrest substituiert sein kann durch eine Aminogruppe, wobei diese Aminogruppe durch zwei gleiche oder verschiedene Substituenten aus der Gruppe Alkyl, Alkoxyalkyl, Aryl oder Aralkyl substituiert ist, oder wobei die Aminogruppe in der Weise substituiert ist, daß 2 Substituenten gemeinsam mit dem Stickstoffatom einen 5- bis 7-gliedrigen Ring bilden, der als weiteres Heteroatom Sauerstoff oder Schwefel oder eine N-Alkyl-Gruppierung enthalten kann,

b) für die Gruppe $SO_2$-$R^5$ steht, wobei $R^5$ einen geradkettigen, verzweigten oder cyclischen gesättigten oder ungesättigten aliphatischen Kohlenwasserstoffrest bedeutet, der gegebenenfalls durch einen Sauerstoff in der Kette unterbrochen ist und der gegebenenfalls substituiert ist durch Phenyl, Phenoxy, Phenylthio, Phenylsulfonyl, Pyridyl, oder Amino, wobei die genannten Arylreste ihrerseits gegebenenfalls substituiert sind durch Halogen, Cyano, Dialkylamino, Alkoxy, Alkyl, Trifluormethyl oder Nitro und wobei die Aminogruppe gegebenenfalls substituiert ist durch zwei gleiche oder

verschiedene Substituenten aus der Gruppe Alkyl, Alkoxyalkyl, Aryl oder Aralkyl, oder wobei 2 dieser Substituenten gegebenenfalls mit dem Stickstoffatom einen 5- bis 7-gliedrigen Ring bilden, der als weiteres Heteroatom Sauerstoff, Schwefel oder die N-Alkyl-Gruppierung enthalten kann,

oder

wobei $R^5$ einen Arylrest bedeutet, der gegebenenfalls 1, 2 oder 3 gleiche oder verschiedene Substituenten der Gruppe Alkyl, Alkoxy, Halogen, Cyano, Trifluormethyl, Trifluormethoxy, Dialkylamino oder Nitro enthält,

c) für die Gruppe $-COR^6$ steht, wobei $R^6$ einen gegebenenfalls substituierten Alkyl-, Aryl- oder Aralkylrest bedeutet,

d) für einen Nitrilrest steht,

und

$R^1$ und $R^2$ gleich oder verschieden sind und jeweils für Wasserstoff, einen geradkettigen oder verzweigten Alkyl-, Aryl- oder einen Aralkylrest stehen

$R^3$ für Wasserstoff oder einen geradkettigen oder verzweigten, gegebenenfalls durch ein Sauerstoffatom unterbrochenen Alkylrest, einen Arylrest oder einen Aralkylrest steht,

sowie ihre pharmazeutisch unbedenklichen Säureadditionssalze.

Es wurde gefunden, daß die ringverbrückten 1.4-Dihydropyridine der allgemeinen Formel I erhalten werden, wenn man

a) 1.4-Dihydropyridine der Formel II, herstellbar nach den für die Synthese von 1.4-Dihydropyridinen üblichen Verfahren,

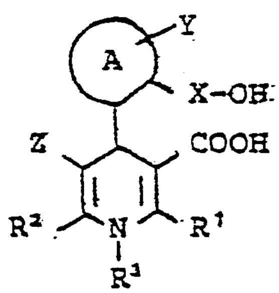

(II),

wobei 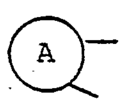 , Y, Z, X, $R^1$, $R^2$, $R^3$ die in Formel (I) angegebene Bedeutung haben,

nach literaturbekannten Methoden zyklisiert;

b) Yliden-ß-ketoester der allgemeinen Formel (III)

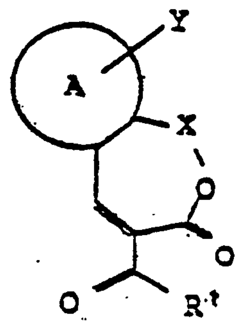

(III),

in welcher 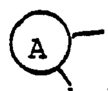 , $R^1$, X und Y die oben angegebene Bedeutung haben,

mit Enaminoverbindungen der allgemeinen Formel (IV)

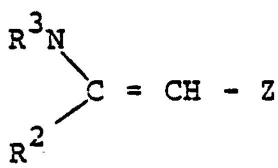

(IV),

in welcher $R^2$, $R^3$ und Z die oben angegebene Bedeutung haben, gegebenenfalls in Gegenwart inerter organischer Lösungsmittel bei Temperaturen zwischen 20 und 150° C umsetzt,

oder

c) Yliden-ß-Ketoester der allgemeinen Formel (III) mit Aminen der allgemeinen Formel (V)

$$R^3 - NH_2 \qquad (V),$$

in welcher $R^3$ die oben angegebene Bedeutung hat

und Ketoverbindungen der allgemeinen Formel (VI)

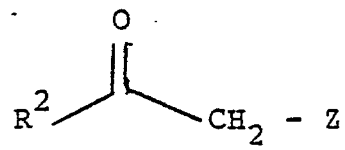 (VI)

in welcher $R^2$ und Z die oben angegebene Bedeutung besitzen, gegebenenfalls in Gegenwart inerter organischer Lösungsmittel bei Temperaturen zwischen 20 und 150° C umsetzt.

Die erfindungsgemäßen ringverbrückten 1,4-Dihydropyridine der allgemeinen Formel I besitzen wertvolle pharmakologische Eigenschaften. Aufgrund ihrer kreislaufbeeinflussenden Wirkung können sie als antihypertensive Mittel, als periphere und cerebrale Vasodilatatoren sowie als Coronartherapeutika Verwendung finden.

Die vorliegende Erfindung betrifft insbesondere 1.4-Dihydropyridine der allgemeinen Formel I, in welcher

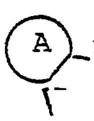 für Phenyl, Naphthyl, Thienyl, Furyl, Pyrryl, Pyridyl, Pyrimidyl, Chinolyl, Isochinolyl, Indolyl, Benzimidazolyl oder Benzoxadiazolyl steht,

Y für einen oder zwei gleiche oder verschiedene Substituenten aus der Gruppe Alkyl, Cycloalkyl, Alkenyl, Alkinyl, Alkoxy mit jeweils bis zu 7 Kohlenstoffatomen, Dioxymethylen, Phenyl, Halogen, -S-Benzyl, O-Benzyl, Trifluormethyl, Trifluormethoxy, Nitro, Cyano, Azido, Hydroxy, Mono- und Dialkylamino mit jeweils 1 bis 4 Kohlenstoffatomen im Alkylrest, Carbonamido, Sulfonamido oder $SO_m$-Alkyl (m=0,1,2; Alkyl bis zu 4 Kohlenstoffatome enthaltend) steht,

X für eine geradkettige oder verzweigte Alkylkette mit bis zu 20 Kohlenstoffatomen steht, die mit 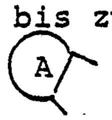 direkt oder über ein Sauerstoff- oder Schwefelatom verknüpft ist und die gegebenenfalls durch ein oder mehrere Sauerstoffatome oder $-\overset{O}{\overset{\|}{C}}-O-$ Gruppen unterbrochen sein kann,

Z a) für die Gruppe $-COOR^4$ steht, wobei $R^4$ für einen geradkettigen, verzweigten oder cyclischen, gesättigten oder ungesättigten Kohlenwasserstoffrest mit bis zu 8 Kohlenstoffatomen steht der gegebenenfalls in der Alkylkette einmal durch Sauerstoff, Schwefel oder die $-SO_2$-Gruppe unterbrochen ist und der gegebenenfalls substituiert ist durch Fluor,

Chlor, Brom, Trifluormethyl oder durch Phenyl, Phenoxy, Phenylthio oder Phenylsulfonyl, wobei die Phenylreste ihrerseits gegebenenfalls ein- oder zweifach substituiert sind durch Fluor, Chlor, Brom, Cyano, Nitro, Trifluormethyl, Alkyl, Alkoxy oder Dialkylamino mit jeweils 1 bis 4 Kohlenstoffatomen in den Alkyl- und Alkoxyresten, oder der Kohlenwasserstoff gegebenenfalls substituiert ist durch eine Aminogruppe, wobei diese Aminogruppe gegebenenfalls zwei gleiche oder verschiedene Substituenten aus der Gruppe Alkyl mit 1 bis 4 Kohlensatoffatomen, Alkoxyalkyl mit bis zu 6 Kohlenstoffatomen, Phenyl, Benzyl oder Phenethyl trägt oder wobei der Stickstoff dieser Aminogruppe mit den Substituenten einen 5- bis 7-gliedrigen Ring bildet, der als weiteres Heteroatom ein Sauerstoff- oder Schwefelatom oder eine N-Alkylgruppe mit 1 bis 4 Kohlenstoffatomen im Alkylrest enthalten kann,

oder

b) für die Gruppe $-SO_2-R^5$ steht, wobei $R^5$ für einen geradkettigen, verzweigten, cyclischen, gesättigten oder ungesättigten aliphatischen Kohlenwasserstoffrest mit bis zu 6 Kohlenstoffatomen steht, der gegebenenfalls durch ein Sauerstoffatom in der Kette unterbrochen ist und der gegebenenfalls substituiert ist durch Phenyl, Phenoxy, Phenylthio oder Phenylsulfonyl, wobei die genannten Phenylreste

ihrerseits gegebenenfalls ein- oder zweifach substituiert sind durch Fluor, Chlor, Brom, Cyano, Nitro, Trifluormethyl, Alkyl, Alkoxy oder Dialkylamino mit jeweils 1 bis 4 Kohlenstoffatomen in den Alkyl- oder Alkoxyresten, oder der Kohlenwasserstoffrest substituiert ist durch eine Aminogruppe, wobei diese Aminogruppe zwei gleiche oder verschiedene Substituenten aus der Gruppe Alkyl, Alkoxyalkyl mit jeweils bis zu 4 Kohlenstoffatomen, Phenyl, Benzyl oder Phenethyl trägt oder wobei die Substituenten dieser Aminogruppe mit dem Stickstoffatom einen 5- bis 7-gliedrigen Ring bilden, der als weiteres Heteroatom ein Sauerstoff- oder Schwefelatom oder die N-Alkylgruppierung enthalten kann, wobei die Alkylgruppe 1 bis 3 Kohlenstoffatome enthält, oder wobei $R^5$ für einen Phenylrest steht, der gegebenenfalls substituiert ist durch 1, 2 oder 3 gleiche oder verschiedene Substituenten aus der Gruppe Nitro, Cyano, Trifluormethyl, Trifluormethoxy, Fluor, Chlor, Brom, Alkyl, Alkoxy und Dialkylamino, wobei die genannten Alkyl- und Alkoxyreste jeweils 1 bis 4 Kohlenstoffatome enthalten,
oder

c) für die Gruppe $-COR^6$ steht, wobei $R^6$ einen geradkettigen oder verzweigten Alkylrest

mit 1 bis 5 Kohlenstoffatomen, Phenyl oder Benzyl bedeutet,

d) für einen Nitrilrest steht,

und

$R^1$ und $R^2$ jeweils gleich oder verschieden sind und für Wasserstoff oder einen geradkettigen oder verzweigten Alkylrest mit 1 bis 4 Kohlenstoffatomen, einen Phenylrest oder Benzylrest stehen

und

$R^3$ für Wasserstoff oder einen geradkettigen oder verzweigten Alkylrest mit 1 bis 6 Kohlenstoffatomen, der gegebenenfalls durch einen Sauerstoff in der Alkylkette unterbrochen ist, oder für einen Phenyl- oder Benzylrest steht,

sowie ihre pharmazeutisch unbedenklichen Säureadditionssalze.

Besonders hervorgehoben seien Verbindungen der allgemeinen Formel (I) in welcher

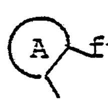 für Phenyl oder Pyridyl steht,

Y für Nitro, Cyano, Trifluormethyl, Trifluormethoxy, Fluor, Chlor, Brom, Azido, Alkoxy mit

bis zu 4 Kohlenstoffatomen oder O-Benzyl steht,

X für eine geradkettige oder verzweigte Alkylkette mit bis zu 12 Kohlenstoffatomen steht, die mit (A) direkt oder über ein Sauerstoff- oder Schwefelatom verknüpft ist und die gegebenenfalls durch ein oder zwei Sauerstoffatome unterbrochen sein kann,

Z a) für die Gruppe $COOR^4$ steht, wobei $R^4$ einen geradkettigen oder verzweigten Alkylrest mit 1 bis 8 Kohlenstoffatomen bedeutet, der gegebenenfalls substituiert durch eine Alkoxygruppe mit bis zu 4 Kohlenstoffatomen, durch eine Cyanogruppe, eine Fluorgruppe, eine Chlorgruppe oder durch eine Aminogruppe, die ihrerseits einmal durch Alkyl mit 1 bis 4 Kohlenstoffatomen substituiert ist und als dritten Substituenten eine identische oder unterschiedliche Alkylgruppe mit 1 bis 4 Kohlenstoffatomen oder einen Benzylrest trägt

oder

b) für einen Nitrilrest steht,

und

$R^1$ und $R^2$ jeweils gleich oder verschieden sind und für Wasserstoff oder Alkyl mit 1 bis 2 Kohlenstoffatomen stehen

und

$R^3$ für Wasserstoff, Alkyl mit bis zu 4 Kohlenstoffatomen oder Benzyl steht,

sowie ihre pharmazeutisch unbedenklichen Säureadditionssalze.

Bei Kenntnis des Standes der Technik war nicht vorhersehbar, daß durch den Ringschluß zwischen Dihydropyridinring und dessen Substituenten in 4-Position Stoffe erhalten werden, die sich durch vorteilhafte biologische Eigenschaften auszeichnen.

Besonders bemerkenswert ist, daß die biologische Wirkung mit der Größe des neu gebildeten Ringes zunimmt.

Aufgrund ihrer unerwarteten pharmazeutischen Wirkung stellen sie eine Bereicherung der Technik dar.

Verfahrensvariante A

Gemäß Verfahrensvariante A werden 1.4-Dihydropyridine der Formel II in Gegenwart von Reagentien, die die Carbonsäurefunktion aktivieren und eine Wasserabspaltung fördern oder die die Alkoholfunktion in eine bessere Abgangsgruppe umwandeln, unter den dafür geeigneten Bedingungen, beispielsweise in hoher Verdünnung, zyklisiert (E. Haslam, Tetrahedron 36, 2409 (1980); K.C. Nicolaou, Tetrahedron 33, 683 (1977); T.G. Back, Tetrahedron 33, 3041 (1977); M. A. Ondetti und P. L. Thomas, J. Am. Chem. Soc. 87, 4373 (1965));

oder

in aktivierte Carbonsäurederivate nach bekannten Verfahren umgewandelt und dann in einem zweiten Schritt gegebenenfalls unter Verwendung eines dafür geeigneten Hilfsstoffs intramolekular mit der am X gebundenen freien Hydroxygruppe zur Reaktion gebracht.

Die als Ausgangsstoffe verwendeteten 1.4-Dihydropyridincarbonsäuren sind neu und können nach bekannten Methoden hergestellt werden (DT-OS 2 847 237).

Von den literaturbekannten Reagenzien zur Aktivierung einer Carbonsäure- oder Alkoholfunktion seien besonders hervorgehoben:

Trifluoressigsäureanhydrid, Quecksilbertrifluoracetat, p-Toluolsulfonylchlorid, 1,1'-Carbonyldiimidazol, 2,2'-Dipyridyldisulfid, 2-Chlor-1-methylpyridiniumjodid, Dicyclohexylcarbodiimid, N-Trimethylsilylimidazol, N,N-Dimethylformamiddineopentylacetal, Azodicarbonsäurediethylester/Triphenylphosphin, 1-Cyclohexyl-3-(2-morpholinoethyl)-carbodiimid-metho-p-toluolsulfonat.

Als Verdünnungsmittel kommen alle geeigneten organischen Lösungsmittel mit Ausnahme von Carbonsäuren und Alkoholen in Frage, vorzugsweise Ether wie Tetrahydrofuran, Diethylether, Dioxan, Glykoldimethylether, Acetonitril, Toluol oder Methylenchlorid.

Verfahrensvariante B

Gemäß Verfahrensvariante B werden Yliden-ß-ketoester der allgemeinen Formel III mit Enaminoverbindungen der allgemeinen Formel IV, gegebenenfalls in Gegenwart inerter organischer Lösungsmittel, vorzugsweise Eisessig, Methanol, Ethanol oder Tetrahydrofuran, umgesetzt.

Verfahrensvariante C

Gemäß Verfahrensvariante C werden Yliden-ß-ketoester der allgemeinen Formel III mit Ketoverbindungen der allgemeinen Formel (VI) und Aminen der allgemeinen Formel (V) gegebenenfalls in Gegenwart inerter organischer Lösungsmittel, beispielsweise Methanol, Ethanol, Isopropanol, Diethylether, Eisessig oder Acetonitril umgesetzt.

Die als Ausgangsstoffe verwendeteten Yliden-ß-ketoester der Formel (III) können nach literaturbekannten Methoden (G. Jones, "The Koevenagel Condensation", in Org. Reactions Vol. XV, 204 ff (1967)) mit darauffolgender intramolekularer Zyklisierung nach den unter Verfahrensvariante A angegebenen Methoden hergestellt werden.

Die als Ausgangsstoffe verwendeten Enaminoverbindungen (IV) sind bekannt oder können nach literaturbekannten Methoden dargestellt werden (vgl. A. C. Cope, J. Am. Chem. Soc. 67, 1017 (1945)).

Die als Ausgangsstoffe verwendeten ß-Ketoverbindungen (VI) sind bekannt oder können nach literaturbekannten Methoden dargestellt werden ( z.B. D.Borrmann, "Umsetzung von Diketen mit Alkoholen, Phenolen und Mercaptanen", in Houben-Weyl, Methoden der organischen Chemie, Vol. VII/4, 230 ff (1968); Y. Oikawa, K. Sugano und O Yonemitsu, J. Org. Chem. 43, 2087 (1978)).

Die Reaktionstemperaturen bei allen Verfahrensvarianten können in einem größeren Bereich variiert werden. Im allgemeinen arbeitet man zwischen 20 und 150° C, vorzugsweise jedoch bei der Siedetemperatur der jeweiligen Lösungsmittels, gelegentlich aber auch bei Temperaturen zwischen +20° und -20° C.

Die Umsetzung kann bei Normaldruck, aber auch bei erhöhtem Druck durchgeführt werden. Im allgemeinen arbeitet man unter Normaldruck.

Die vorstehenden Herstellungsverfahren sind lediglich zur Verdeutlichung angegeben, und die Herstellung der Verbindungen der Formel(I) ist nicht auf diese Verfahren beschränkt, sondern jede Modifikation dieser Verfahren ist in gleicher Weise für die Herstellung der erfindungsgemäßen Verbindungen anwendbar.

Die erfindungsgemäßen Verbindungen können in stereoisomeren Formen existieren, die sich entweder wie Bild und Spiegelbild (Enantiomere) oder die sich nicht wie Bild und Spiegelbild (Diastereomere) verhalten.

Sowohl die Antipoden als auch die Diastereomerengemische sind Gegenstand der vorliegenden Erfindung. Die Racemformen lassen sich ebenso wie die Diastereomeren in bekannter Weise in die stereoisomer einheitlichen Bestandteile trennen (vgl. z.B. E.L. Eliel, Stereochemistry of Carbon Compounds, McGraw Hill, 1962).

Als Beispiele erfindungsgemäßer Verbindungen seien genannt:

1.4-Dihydro-2.6-dimethyl-4-(2-(5-hydroxypentoxy)phenyl)-pyridin-3-butoxycarbonyl-5-carbonsäurelacton,
1.4-Dihydro-2.6-dimethyl-4-(2-(3-hydroxypropoxy)-3-nitrophenyl)-pyridin-3-isopropyloxycarbonyl-5-carbonsäurelacton,
1.4-Dihydro-2.6-dimethyl-4-(2-(4-hydroxybutoxy)-5-nitrophenyl)-pyridin-3-oktyloxycarbonyl-5-carbonsäurelacton,
1.4-Dihydro-2.6-dimethyl-4-(2-(3-hydroxypropoxy)-3-methoxyphenyl)-pyridin-3-butoxycarbonyl-5-carbonsäurelacton,
1.4-Dihydro-2.6-dimethyl-4-(2-(4-hydroxybutoxy)-5-chlorphenyl)-pyridin-3-methoxycarbonyl-5-carbonsäurelacton,
1.4-Dihydro-2.6-dimethyl-4-(2-(hydroxyethyl)phenyl)-pyridin-3-propoxycarbonyl-5-carbonsäurelacton,

1.4-Dihydro-2.6-dimethyl-4-(2-(11-hydroxyundecyloxy)phenyl)-pyridin-3-oktyloxycarbonyl-5-carbonsäurelacton

1.4-Dihydro-2.6-dimethyl-4-(2-(4-hydroxybutoxy)phenyl)-pyridin-3-methoxycarbonyl-5-carbonsäurelacton.

Die Verbindungen der allgemeinen Formel (I) zeigen interessante biologische Wirkungen. Sie besitzen ein breites und vielseitiges pharmakologisches Wirkungsspektrum. Im einzelnen seien folgende Hauptwirkungen genannt:

1. Die Verbindungen bewirken bei parenteraler, oraler und perlingualer Zugabe eine deutliche Erweiterung der Coronargefäße. Diese Wirkung auf die Coronargefäße wird durch einen gleichzeitigen nitritähnlichen herzentlastenden Effekt verstärkt.

   Sie beeinflussen bzw. verändern den Herzstoffwechsel im Sinne einer Energieersparnis.

2. Die Erregbarkeit des Reizbildungs- und Erregungsleitungssystems innerhalb des Herzens wird herabgesetzt, so daß eine in therapeutischen Dosen nachweisbare Antiflimmerwirkung resultiert.

3. Der Tonus der glatten Muskulatur der Gefäße wird unter der Wirkung der Verbindungen stark vermindert. Diese gefäßspasmolytische Wirkung kann im gesamten Gefäßsystem stattfinden, oder sich mehr oder weniger isoliert in umschriebenen Gefäßgebieten (wie z.B. im Gehirn) manifestieren.

4. Die Verbindungen senken den Blutdruck von normotonen und hypertonen Tieren und können somit als antihypertensive Mittel verwendet werden.

5. Die Verbindungen haben stark muskulär-spasmolytische Wirkungen, die an der glatten Muskulatur des Magens, Darmtraktes, des Urogenitaltraktes und des Respirationssystems deutlich werden.

Die neuen Wirkstoffe können in bekannter Weise in die üblichen Formulierungen überführt werden, wie Tabletten, Kapseln, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösungsmittel.
Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt

durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:

Wasser, nicht-toxische organische Lösungsmittel, wie Paraffine (z.B. Erdölfraktionen), pflanzliche Öle (z.B. Erdnuß-/Sesamöl), Alkohole (z.B. Ethylalkohol, Glycerin), Glykole (z.B. Propylenglykol, Polyethylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Roh-, Milch- und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin-Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixiere, die für orale Anwendung gedacht sind, können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Anwendung Mengen von etwa 0,01 bis 10 mg/kg, vorzugsweise etwa 0,01 bis 5 mg/kg Körpergewicht pro Tag zur Erzielung wirksamer Ergebnisse zu verabreichen, und bei oraler Applikation beträgt die Dosierung etwa 0,1 bis 50 mg/kg, vorzugsweise 0,5 bis 10 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. der Art von dessen Formulierung und dem Zeitpunkt bzw. Intervall, zu

welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

## Herstellungsbeispiele

### Beispiel 1:

Darstellung von 1.4-Dihydro-2.6-dimethyl-4-(2-(2-hydroxyethoxy)phenyl)-pyridin-3-ethyloxycarbonyl-5-carbonsäurelacton.

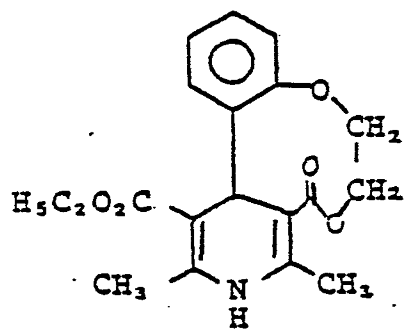

Zu einer Suspension von 10 g (23.6 mmol) 1-Cyclohexyl-3-(2-morpholinoethyl)carbodiimidmetho-p-toluolsulfonat in 200 ml abs. THF werden 1.5 g (12 mmol) 4-N,N-Dimethylaminopyridin zugegeben und darauf unter Stickstoff innerhalb von 72 h eine Lösung von 5 g (13.3 mmol) 1.4-Dihydro-2.6-dimethyl-4-(2-(2-hydroxyethoxy-phenyl)-pyridin-3-ethyloxycarbonyl-5-carbonsäure in 500 ml abs. THF zugetropft. Man filtriert den entstandenen Harnstoff und überschüssiges Reagenz ab, destilliert das Lösungsmittel im Vakuum ab und kristallisiert aus Ethanol um oder reinigt durch Chromatographie.

Schmelzpunkt: 213°C Ausbeute: 1.90 g (30%).

Analog Beispiel 1 werden hergestellt:

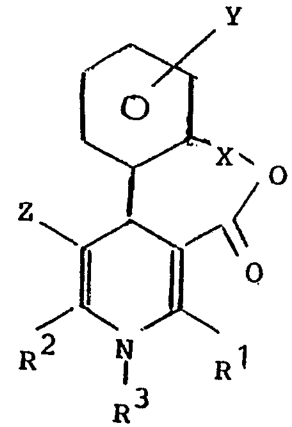

| Bsp. | X | Y | Z | $R^1$ | $R^2$ | $R^3$ | Fp: | Ausbeute |
|---|---|---|---|---|---|---|---|---|
| 2 | 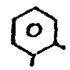 $O(CH_2)_3-$ | -H | $-CO_2C_2H_5$ | $-CH_3$ | $-CH_3$ | -H | 186° | 28 % |
| 3 | 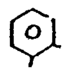 $O(CH_2)_4-$ | -H | $-CO_2C_2H_5$ | $-CH_3$ | $-CH_3$ | -H | 165° | 5 % |
| 4 | 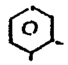 $O(CH_2)_{11}-$ | -H | $-CO_2C_2H_5$ | $-CH_3$ | $-CH_3$ | -H | amorph | 9 % |
| 5 | 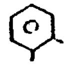 $CH_2-$ | -H | $-CO_2C_2H_5$ | $-CH_3$ | $-CH_3$ | -H | 203° | 39 % |
| 6 | 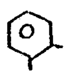 $CH_2-CH_2-$ | -H | $-CO_2C_2H_5$ | $-CH_3$ | $-CH_3$ | -H | 196° | 49 % |
| 7 | 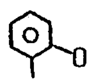 $O(CH_2)_5-$ | -H | $-CO_2C_2H_5$ | $-CH_3$ | $-CH_3$ | -H | 169° | 60 % |

Patentansprüche:

1. 1.4-Dihydropyridine der allgemeinen Formel I

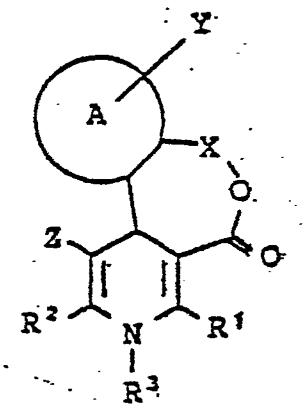

(I),

in welcher

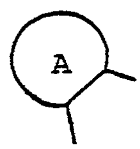

für Aryl, Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Chinolyl, Isochinolyl, Indolyl, Benzimidazolyl, Benzoxadiazolyl, Chinazolyl oder Chinoxalyl steht,

Y für einen oder zwei gleiche oder verschiedene Substituenten aus der Gruppe Alkyl, Alkenyl, Alkinyl, Alkoxy, Alkenoxy, Aryloxy, Aralkoxy, Alkinoxy, Phenyl, Halogen, Alkylen, Dioxyalkylen, Trifluormethyl, Trifluormethoxy, Nitro, Cyano, Azido, Carboxy, Hydroxy, Amino, Alkylamino, Halogenalkoxy, Carbalkoxy,

Carbonamido, Sulfonamido, $SO_m$-Alkyl, $SO_m$-Aryl oder $SO_m$-Aralkyl (m=0,1,2) steht,

X für eine geradkettige oder verzweigte Alkylkette mit bis zu 20 Kohlenstoffatomen steht, die mit 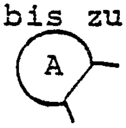 direkt oder über ein Sauerstoff-, Schwefel- oder Stickstoffatom verknüpft ist und die gegebenenfalls durch ein oder mehrere Sauerstoffatome, Stickstoffatome oder $-\overset{O}{\overset{\|}{C}}-O-$ Gruppen unterbrochen sein kann, wobei die oben genannten Stickstoffatome durch H, Alkyl, Acyl oder Benzyl substituiert sein können,

Z a) für die Gruppe $-COOR^4$ steht, wobei $R^4$ für einen geradkettigen, verzweigten oder cyclischen, gesättigten oder ungesättigten Kohlenwasserstoff steht, der durch Sauerstoff, Schwefel oder die $-SO_2$-Gruppe in der Kette unterbrochen sein kann, und der gegebenenfalls substituiert ist durch Halogen, Cyano, Pyridyl, Phenyl, Phenoxy, Phenylthio oder Phenylsulfonyl, wobei die Phenylgruppen ihrerseits substituiert sein können durch Halogen, Cyano, Dialkylamino, Alkoxy, Alkyl, Trifluormethyl oder Nitro, oder

wobei der Kohlenwasserstoffrest substituiert sein kann durch eine Aminogruppe, wobei diese Aminogruppe durch zwei gleiche oder verschiedene Substituenten aus der Gruppe Alkyl, Alkoxyalkyl, Aryl oder Aralkyl substituiert ist, oder wobei die Aminogruppe in der Weise substituiert ist, daß 2 Substituenten gemeinsam mit dem Stickstoffatom einen 5- bis 7-gliedrigen Ring bilden, der als weiteres Heteroatom Sauerstoff oder Schwefel oder eine N-Alkyl-Gruppierung enthalten kann,

b) für die Gruppe $SO_2-R^5$ steht, wobei $R^5$ einen geradkettigen, verzweigten oder cyclischen gesättigten oder ungesättigten aliphatischen Kohlenwasserstoffrest bedeutet, der gegebenenfalls durch einen Sauerstoff in der Kette unterbrochen ist und der gegebenenfalls substituiert ist durch Phenyl, Phenoxy, Phenylthio, Phenylsulfonyl, Pyridyl, oder Amino, wobei die genannten Arylreste ihrerseits gegebenenfalls substituiert sind durch Halogen, Cyano, Dialkylamino, Alkoxy, Alkyl, Trifluormethyl oder Nitro und wobei die Aminogruppe gegebenenfalls substituiert ist durch zwei gleiche oder

verschiedene Substituenten aus der Gruppe Alkyl, Alkoxyalkyl, Aryl oder Aralkyl, oder wobei 2 dieser Substituenten gegebenenfalls mit dem Stickstoffatom einen 5- bis 7-gliedrigen Ring bilden, der als weiteres Heteroatom Sauerstoff, Schwefel oder die N-Alkyl-Gruppierung enthalten kann,

oder

wobei $R^5$ einen Arylrest bedeutet, der gegebenenfalls 1, 2 oder 3 gleiche oder verschiedene Substituenten der Gruppe Alkyl, Alkoxy, Halogen, Cyano, Trifluormethyl, Trifluormethoxy, Dialkylamino oder Nitro enthält.

c) für die Gruppe $-COR^6$ steht, wobei $R^6$ einen gegebenenfalls substituierten Alkyl-, Aryl- oder Aralkylrest bedeutet,

d) für einen Nitrilrest steht,

und

$R^1$ und $R^2$ gleich oder verschieden sind und jeweils für Wasserstoff, einen geradkettigen oder verzweigten Alkyl-, Aryl- oder einen Aralkylrest stehen

$R^3$ für Wasserstoff oder einen geradkettigen oder verzweigten, gegebenenfalls durch ein Sauerstoffatom unterbrochenen Alkylrest, einen Arylrest oder einen Aralkylrest steht,

sowie ihre pharmazeutisch unbedenklichen Säureadditionssalze.

2. 1.4-Dihydropyridine nach Anspruch 1 der allgemeinen Formel I, in welcher

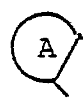 für Phenyl, Naphthyl, Thienyl, Furyl, Pyrryl, Pyridyl, Pyrimidyl, Chinolyl, Isochinolyl, Indolyl, Benzimidazolyl oder Benzoxadiazolyl steht,

Y für einen oder zwei gleiche oder verschiedene Substituenten aus der Gruppe Alkyl, Cycloalkyl, Alkenyl,Alkinyl, Alkoxy mit jeweils bis zu 7 Kohlenstoffatomen, Dioxymethylen, Phenyl, Halogen, -S-Benzyl, O-Benzyl, Trifluormethyl, Trifluormethoxy, Nitro, Cyano, Azido, Hydroxy, Mono- und Dialkylamino mit jeweils 1 bis 4 Kohlenstoffatomen im Alkylrest, Carbonamido, Sulfonamido oder $SO_m$-Alkyl (m=0,1,2; Alkyl bis zu 4 Kohlenstoffatome enthaltend) steht,

X für eine geradkettige oder verzweigte Alkylkette mit bis zu 20 Kohlenstoffatomen steht, die mit 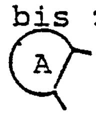 direkt oder über ein Sauerstoff- oder Schwefelatom verknüpft ist und die gegebenenfalls durch ein oder mehrere Sauerstoffatome oder

$-\overset{\overset{O}{\|}}{C}-O-$ Gruppen unterbrochen sein kann,

Z a) für die Gruppe $-COOR^4$ steht, wobei $R^4$ für einen geradkettigen, verzweigten oder cyclischen, gesättigten oder ungesättigten Kohlenwasserstoffrest mit bis zu 8 Kohlenstoffatomen steht der gegebenenfalls in der Alkylkette einmal durch Sauerstoff, Schwefel oder die $-SO_2$-Gruppe unterbrochen ist und der gegebenenfalls substituiert ist durch Fluor, Chlor, Brom, Trifluormethyl oder durch Phenyl, Phenoxy, Phenylthio oder Phenylsulfonyl, wobei die Phenylreste ihrerseits gegebenenfalls ein- oder zweifach substituiert sind durch Fluor, Chlor, Brom, Cyano, Nitro, Trifluormethyl, Alkyl, Alkoxy oder Dialkylamino mit jeweils 1 bis 4 Kohlenstoffatomen in den Alkyl- und Alkoxyresten, oder der Kohlenwasserstoff gegebenenfalls substituiert ist durch eine Aminogruppe, wobei diese Aminogruppe gegebenenfalls zwei gleiche oder verschiedene Substituenten aus der Gruppe Alkyl mit 1 bis 4 Kohlenstoffatomen, Alkoxyalkyl mit bis zu 6 Kohlenstoffatomen, Phenyl, Benzyl oder Phenethyl trägt oder wobei der Stickstoff dieser Aminogruppe mit den Substituenten einen 5- bis 7-gliedrigen Ring bildet, der als weiteres Heteroatom ein Sauerstoff- oder Schwefelatom oder

eine N-Alkylgruppe mit 1 bis 4 Kohlenstoffatomen im Alkylrest enthalten kann,

oder

b) für die Gruppe $-SO_2-R^5$ steht, wobei $R^5$ für einen geradkettigen, verzweigten, cyclischen, gesättigten oder ungesättigten aliphatischen Kohlenwasserstoffrest mit bis zu 6 Kohlenstoffatomen steht, der gegebenenfalls durch ein Sauerstoffatom in der Kette unterbrochen ist und der gegebenenfalls substituiert ist durch Phenyl, Phenoxy, Phenylthio oder Phenylsulfonyl, wobei die genannten Phenylreste ihrerseits gegebenenfalls ein- oder zweifach substituiert sind durch Fluor, Chlor, Brom, Cyano, Nitro, Trifluormethyl, Alkyl, Alkoxy oder Dialkylamino mit jeweils 1 bis 4 Kohlenstoffatomen in den Alkyl- oder Alkoxyresten, oder der Kohlenwasserstoffrest substituiert ist durch eine Aminogruppe, wobei diese Aminogruppe zwei gleiche oder verschiedene Substituenten aus der Gruppe Alkyl, Alkoxyalkyl mit jeweils bis zu 4 Kohlenstoffatomen, Phenyl, Benzyl oder Phenethyl trägt oder wobei die Substituenten dieser Aminogruppe mit dem Stickstoffatom einen 5- bis 7-gliedrigen Ring bilden, der als weiteres Heteroatom ein Sauerstoff- oder Schwefelatom oder die N-Alkylgruppierung enthalten kann, wobei die Alkylgruppe 1 bis 3 Kohlenstoffatome enthält, oder

wobei $R^5$ für einen Phenylrest steht, der gegebenenfalls substituiert ist durch 1, 2 oder 3 gleiche oder verschiedene Substituenten aus der Gruppe Nitro, Cyano, Trifluormethyl, Trifluormethoxy, Fluor, Chlor, Brom, Alkyl, Alkoxy und Dialkylamino, wobei die genannten Alkyl- und Alkoxyreste jeweils 1 bis 4 Kohlenstoffatome enthalten,
oder

c) für die Gruppe $-COR^6$ steht, wobei $R^6$ einen geradkettigen oder verzweigten Alkylrest mit 1 bis 5 Kohlenstoffatomen, Phenyl oder Benzyl bedeutet,

d) für einen Nitrilrest steht,

und

$R^1$ und $R^2$ jeweils gleich oder verschieden sind und für Wasserstoff oder einen geradkettigen oder verzweigten Alkylrest mit 1 bis 4 Kohlenstoffatomen, einen Phenylrest oder Benzylrest stehen

und

$R^3$ für Wasserstoff oder einen geradkettigen oder verzweigten Alkylrest mit 1 bis 6 Kohlenstoffatomen, der gegebenenfalls durch einen Sauerstoff in der Alkylkette

unterbrochen ist, oder für einen Phenyl- oder Benzylrest steht,

sowie ihre pharmazeutisch unbedenklichen Säureadditionssalze.

3. 1.4-Dihydropyridine nach Anspruch 1 der allgemeinen Formel (I) in welcher

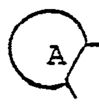 für Phenyl oder Pyridyl steht,

Y für Nitro, Cyano, Trifluormethyl, Trifluormethoxy, Fluor, Chlor, Brom, Azido, Alkoxy mit bis zu 4 Kohlenstoffatomen oder O-Benzyl steht,

X für eine geradkettige oder verzweigte Alkylkette mit bis zu 12 Kohlenstoffatomen steht, die mit 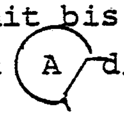 direkt oder über ein Sauerstoff- oder Schwefelatom verknüpft ist und die gegebenenfalls durch ein oder zwei Sauerstoffatome unterbrochen sein kann,

Z a) für die Gruppe $COOR^4$ steht, wobei $R^4$ einen geradkettigen oder verzweigten Alkylrest mit 1 bis 8 Kohlenstoffatomen bedeutet, der gegebenenfalls substituiert durch eine Alkoxygruppe mit bis zu 4 Kohlenstoffatomen, durch eine Cyanogruppe, eine Fluorgruppe, eine Chlorgruppe oder durch eine

Aminogruppe, die ihrerseits einmal durch Alkyl mit 1 bis 4 Kohlenstoffatomen substituiert ist und als dritten Substituenten eine identische oder unterschiedliche Alkylgruppe mit 1 bis 4 Kohlenstoffatomen oder einen Benzylrest trägt

oder

b) für einen Nitrilrest steht,

und

$R^1$ und $R^2$ jeweils gleich oder verschieden sind und für Wasserstoff oder Alkyl mit 1 bis 2 Kohlenstoffatomen stehen

und

$R^3$ für Wasserstoff, Alkyl mit bis zu 4 Kohlenstoffatomen oder Benzyl steht,

sowie ihre pharmazeutisch unbedenklichen Säureadditionssalze.

4. Verfahren zur Herstellung von 1.4-Dihydropyridinen der allgemeinen Formel (I)

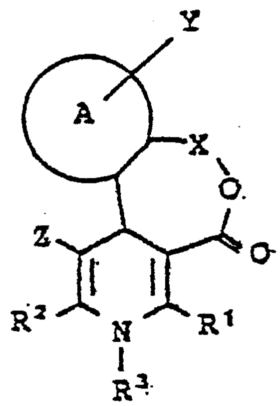

(I),

in welcher

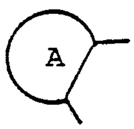 für Aryl, Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Chinolyl, Isochinolyl, Indolyl, Benzimidazolyl, Benzoxadiazolyl, Chinazolyl oder Chinoxalyl steht,

Y für einen oder zwei gleiche oder verschiedene Substituenten aus der Gruppe Alkyl, Alkenyl, Alkinyl, Alkoxy, Alkenoxy, Aryloxy, Aralkoxy, Alkinoxy, Phenyl, Halogen, Alkylen, Dioxyalkylen, Trifluormethyl, Trifluormethoxy, Nitro, Cyano, Azido, Carboxy, Hydroxy, Amino, Alkylamino, Halogenalkoxy, Carbalkoxy,

Carbonamido, Sulfonamido, $SO_m$-Alkyl, $SO_m$-Aryl oder $SO_m$-Aralkyl (m=0,1,2) steht,

X für eine geradkettige oder verzweigte Alkylkette mit bis zu 20 Kohlenstoffatomen steht, die mit 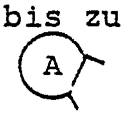 direkt oder über ein Sauerstoff-, Schwefel- oder Stickstoffatom verknüpft ist und die gegebenenfalls durch ein oder mehrere Sauerstoffatome, Stickstoffatome oder $-\overset{O}{\overset{\|}{C}}-O-$ Gruppen unterbrochen sein kann, wobei die oben genannten Stickstoffatome durch H, Alkyl, Acyl oder Benzyl substituiert sein können,

Z a) für die Gruppe $-COOR^4$ steht, wobei $R^4$ für einen geradkettigen, verzweigten oder cyclischen, gesättigten oder ungesättigten Kohlenwasserstoff steht, der durch Sauerstoff, Schwefel oder die $-SO_2$-Gruppe in der Kette unterbrochen sein kann, und der gegebenenfalls substituiert ist durch Halogen, Cyano, Pyridyl, Phenyl, Phenoxy, Phenylthio oder Phenylsulfonyl, wobei die Phenylgruppen ihrerseits substituiert sein können durch Halogen, Cyano, Dialkylamino, Alkoxy, Alkyl, Trifluormethyl oder Nitro, oder

wobei der Kohlenwasserstoffrest substituiert sein kann durch eine Aminogruppe, wobei diese Aminogruppe durch zwei gleiche oder verschiedene Substituenten aus der Gruppe Alkyl, Alkoxyalkyl, Aryl oder Aralkyl substituiert ist, oder wobei die Aminogruppe in der Weise substituiert ist, daß 2 Substituenten gemeinsam mit dem Stickstoffatom einen 5- bis 7-gliedrigen Ring bilden, der als weiteres Heteroatom Sauerstoff oder Schwefel oder eine N-Alkyl-Gruppierung enthalten kann,

b) für die Gruppe $SO_2$-$R^5$ steht, wobei $R^5$ einen geradkettigen, verzweigten oder cyclischen gesättigten oder ungesättigten aliphatischen Kohlenwasserstoffrest bedeutet, der gegebenenfalls durch einen Sauerstoff in der Kette unterbrochen ist und der gegebenenfalls substituiert ist durch Phenyl, Phenoxy, Phenylthio, Phenylsulfonyl, Pyridyl, oder Amino, wobei die genannten Arylreste ihrerseits gegebenenfalls substituiert sind durch Halogen, Cyano, Dialkylamino, Alkoxy, Alkyl, Trifluormethyl oder Nitro und wobei die Aminogruppe gegebenenfalls substituiert ist durch zwei gleiche oder

verschiedene Substituenten aus der Gruppe Alkyl, Alkoxyalkyl, Aryl oder Aralkyl, oder wobei 2 dieser Substituenten gegebenenfalls mit dem Stickstoffatom einen 5- bis 7-gliedrigen Ring bilden, der als weiteres Heteroatom Sauerstoff, Schwefel oder die N-Alkyl-Gruppierung enthalten kann,

oder

wobei $R^5$ einen Arylrest bedeutet, der gegebenenfalls 1, 2 oder 3 gleiche oder verschiedene Substituenten der Gruppe Alkyl, Alkoxy, Halogen, Cyano, Trifluormethyl, Trifluormethoxy, Dialkylamino oder Nitro enthält.

c) für die Gruppe $-COR^6$ steht, wobei $R^6$ einen gegebenenfalls substituierten Alkyl-, Aryl- oder Aralkylrest bedeutet,

d) für einen Nitrilrest steht,

und

$R^1$ und $R^2$ gleich oder verschieden sind und jeweils für Wasserstoff, einen geradkettigen oder verzweigten Alkyl-, Aryl- oder Aralkylrest stehen,

$R^3$ für Wasserstoff oder einen geradkettigen oder verzweigten, gegebenenfalls durch ein Sauerstoffatom unterbrochenen Alkylrest, einen Arylrest oder einen Aralkylrest steht, dadurch gekennzeichnet, daß man

a) 1.4-Dihydropyridine der Formel II, herstellbar nach den für die Synthese von 1.4-Dihydropyridinen üblichen Verfahren,

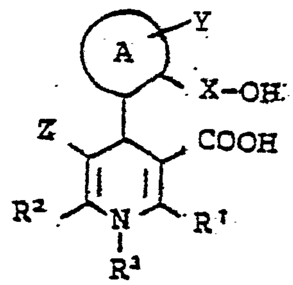

(II),

wobei 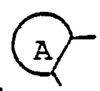 , Y, Z, X, $R^1$, $R^2$, $R^3$ die in Formel (I) angegebene Bedeutung haben, nach literaturbekannten Methoden zyklisiert,

b) Yliden-ß-ketoester der allgemeinen Formel (III)

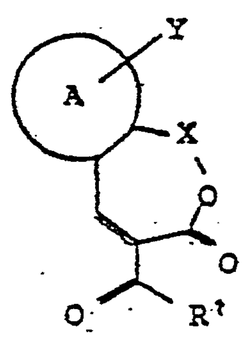

(III),

in welcher 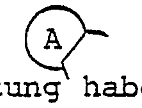 , $R^1$, X und Y die oben angegebene Bedeutung haben,

mit Enaminoverbindungen der allgemeinen Formel (IV)

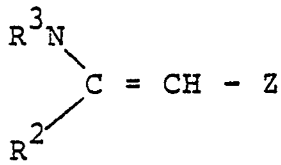

(IV),

in welcher $R^2$, $R^3$ und Z die oben angegebene Bedeutung haben, gegebenenfalls in Gegenwart inerter organischer Lösungsmittel bei Temperaturen zwischen 20 und 150° C umsetzt,

oder

c) Yliden-ß-Ketoester der allgemeinen Formel (III) mit Aminen der allgemeinen Formel (V)

$$R^3 - NH_2 \qquad (V),$$

in welcher $R^3$ die angebene Bedeutung hat

und Ketoverbindungen der allgemeinen Formel (VI)

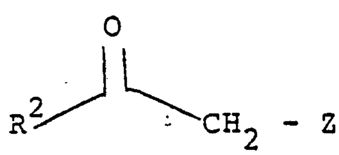

(VI)

in welcher $R^2$ und Z die oben angegebene Bedeutung besitzen, gegebenenfalls in Gegenwart inerter organischer Lösungsmittel bei Temperaturen zwischen 20 und 150° C umsetzt.

5. Arzneimittel enthaltend mindestens eine Verbindung der allgemeinen Formel I gemäß Anspruch 1.

6. Verfahren zur Herstellung von Arzneimitteln, dadurch gekennzeichent, daß man Verbindungen der allgemeinen Formel I gemäß Anspruch 1 gegebenenfalls unter Verwendung von üblichen Hilfs- und Trägerstoffen in eine geeignete Applikationsform überführt.

7. Verwendung von Verbindungen der allgemeinen Formel I gemäß Anspruch 1 bei der Bekämpfung von Erkrankungen.

8. Verwendung von Verbindungen der allgemeinen Formel I gemäß Anspruch 1 bei der Bekämpfung von Kreislauferkrankungen.